# EUROPEAN PATENT APPLICATION

(11) **EP 0 680 728 A1**
(43) Date of publication of application: **08.11.1995**
(21) Application number: 95830158.2
(22) Date of filing: 20.04.1995
(51) Int. Cl.: A61B 5/18

(54) **System for the automatic monitoring of the level of the attention of a human operator**

(30) Priority: 04.05.1994 IT RM940279
(71) Applicant: Alisea - Assistenza Logistica e Ingegneria per i Sistemi Elettronici e d'Automazione Srl, I-74100 Taranto (IT)
(72) Inventor: Cioce, Guiseppe, I-70054 Giovinazzo (BA) (IT)

(57) **Abstract**

The invention concerns a system able to continuosly measure the psycho-physical condition of a human operator, with the purpose of detecting possible decrease of his level of attention or vigilance.

The invention, in a form of a presently preferred specific realization, illustrated by FIGURE 1, is characterized by the following specification:
A **Control Unit** (1) exploiting a time base supplied by a **Clock** (2), randomly draws the attention of the operator by means of the activation of an **Alarm Unit** (3) and measures, by means of a **Timer** (4), the time elapsed until the operator gives back an answer by enabling one of n **Response Circuits** (5a, 5b, ..... 5n), being the operator response detected by a **Detector** (6), being the duration of the time interval between the Alarm indicator (3) activation and the Response Circuit (5a, 5b,..... 5n) response compared with the duration data previously stored in a **Memory** (7) and being the Control Unit (1) able to generate an visual or acoustic signal by means of the Alarm Unit (3) and able to activate an **Actuator** (8) in the case the measured duration is greater than a preset threshold.

The system could be exploited when an operator, committed to a critical operation, is requested to maintain a persistent state of vigilance i.e.: driving of vehicles, command and control of dangerous processes, watch or guard services, etc..

## Description

### Technical field to wich the invention relates

The invention concerns a system able to continously measure the psychophysical condition of an human operator, with the purpose of detecting possible decrease in his level of attention or vigilance.

On the basis of our knowledge, the systems for monitoring the level of attention of an operator, are used only in the case of railway transportation. These systems request that the operator react, within a certain time, activating a push button or a pedal, in response to a sound signal that is generated at constant time intervals (i.e. each 3 minuts). In the case of a missed reply, the system enables an sound alarm signal and can, if requested, activate external actuators for preventing possible dangerous conditions.

The above mentioned technique is not suitable for situations different from the railway transportation, because it might divert the operator by requiring unexpected actions that can interfere with the ordinary procedures. Further more, because of the rigid nature of the above mentioned tecniques (constant frequency of the stimulus), it can not match the current psycho-physical condition of the operator.

### Summary of the invention

The primary objective of this invention is to provide systems for continously monitoring the psycho-physical condition of an operator, by recurrent interrogations, generated with a frequency that is adjusted according to the parameter values directly related to the current level of the operator's attention.

The secondary purpose of the invention is to provide systems which, in case of critical levels of attention are detected, enables specific actuators in order to remove the potential negative consequences of the reduced attention.

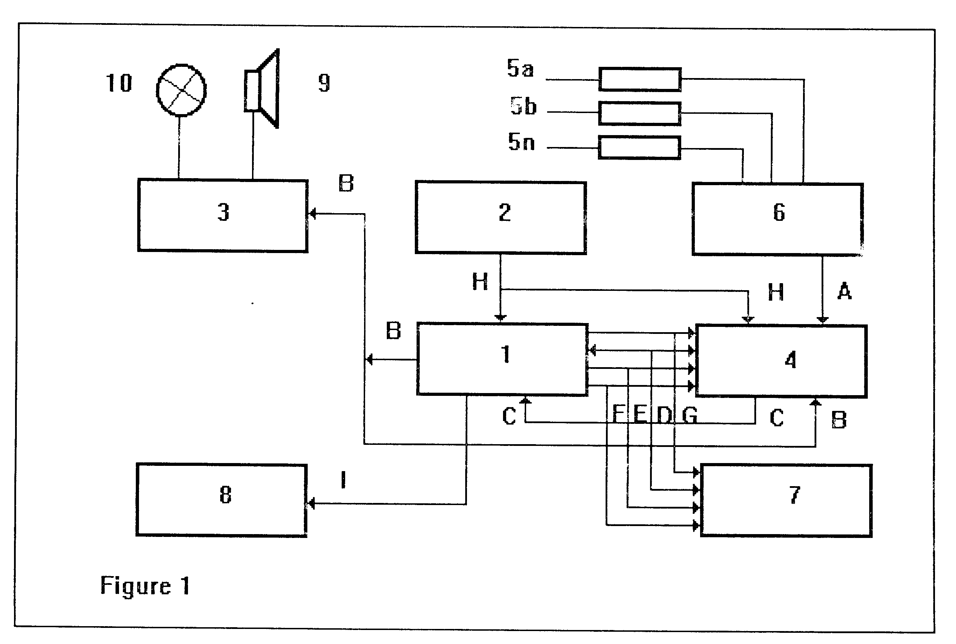

The invention, in a form of a presently preferred specific realization, illustrated by FIGURE 1, is characterized by the following specification:
A **Control Unit** (1) exploiting a time base supplied by a **Clock** (2), randomly draws the attention of the operator by means of the activation of an **Alarm Unit** (3) and measures, by means of a **Timer** (4), the time elapsed until the operator gives back an answer by enabling one of n **Response Circuits** (5a, 5b, ..... 5n).

The operator response is detected by a **Detector** (6), and the duration of the time interval between the activation of the Alarm Indicator (3) and the response of the Response Circuits (5a, 5b, ..... 5n), compared with the duration data previously stored in a **Memory** (7) enables the Control Unit (1) to generate an visual or acoustic signal by means of the Alarm Unit (3) and to activate an **Actuator** (8) in case the measured duration is greater than a preset threshold.

### Detailed description of possible designs of the system

The invention is herein descripted on the base of the attached Figure 1, with reference to a present preferred realization, which is reported with the sole purpose of exeplification without any restriction for different implementations.

The attached Figure 1: Schematic diagram of the principle of the System for the automatic detection and measurement of the psycho-physcal level of the attention of a human operator, includes the following designators:
**(1) Control Unit** - a device capable of generating response requests to an operator, with a frequency adjusted to the psicho-phisical condition of the operator himself and capable of measuring and storing, by means respectively of the Timer (4) and the Memory (7) the respose time of the operator.
**(2) Clock** - a device able to generate clock signals, used as time reference by the Control Unit (1).
**(3) Alarm Unit** - a device that generate, when enabled by the Control Unit (1), alarm signals which activate a Loudspeaker (9) and/or a Visual Indicator (10), in order to recall the attention of the operator, without interfering with his ordinary tasks.
**(4) Timer** - a device able to measure the time intervals between the response requests sent to the operator by the Control Unit (1) by means of the Alarm Unit (3) that generate alarm signals that pilot the Loudspeaker (9) and/or the Visual Indicator (10) and the response provided by the operator himself through the activation of one of the n Response Circuits (5a, 5b, .... 5n).
**(5) Response Circuits** - one of n circuits, the activation of which, is interpreted as the response of the operator to the request of the Control Unit (1).
**(6) Detector** - a device able to detect the activation of one of the Response Circuits (5).
   This device can interpretate as operator's response, either the activation of a dedicated response circuit or the activation of one of the circuits that the operator can activate during his ordinary tasks and designates as Response Circuits (5a, 5b, .... 5n).
**(7) Memory** - a storing device exploited by the Control Unit (1) for the storage of the time intervals measured by the Timer (4).
**(8) Actuator** - a device able to provide specific actions in order to prevent the consequences of the reduced level of attention of the operator. The device and its preventing action will depend on the nature of the current operations.
**(9) Loudspeaker -** a sound transducer for generating sound alarms.
**(10) Visual Indicator -** a device used for generating visual alarms.

With reference to the attached Figure 1 that shows the basic schematic diagram of the System for the automatic detection and measurement of the psycho-physcal level of the attention of a human operator, the principle of operation of the invention can be described as follows:

At the system initialization, the Control Unit (1), after having set to zero the content of the Memory (7), initiates a "calibration" cycle, by measuring a series of response times of the operator. To this end, the Control Unit, exploiting as time base the signal supplied by the Clock (2), repetitively activates (i.e. every 10 seconds) the Alarm Unit (3), requesting that the operator answer by activating one of the possible Response Circuits (5a, 5b, .... 5n).

The Detector (6), which is connected to the Response Circuits (5a,5b, ... 5n), constantly surveys the voltage or the current levels of the circuits and, in case of change of those parameters, it interprets the phenomenon as an operator response and consequently supplies a signal (A) to the Timer (4).

The Timer (4) collects the signals (B) which the Control Unit (1) also sends to the Alarm Unit (3) and collects the signals (A) generated by the Detector (6) every time it detects a change of the voltage or current in the Response Circuits (5a, 5b, ... 5n). By comparing the time of arrival of the two signals, the Timer (4) determines the operator's response time and supplies it, in the form of signal (C), to the Controller (1).

On the basis of the operator's response times, the Controller (1) sets the initial interrogation time rate (C0) and the initial threshold (S0).

After the time (C0) has elapsed from the last interrogation, the Control Unit (1) generates a new request for the operator's response. The Control Unit (1) collects the new operator's reponse time after a time T1 (generally lower than the initial threshold (S0)) and makes the Memory (7) replace with the new time T1 the oldest of the previously stored time values; the Control Unit (1), on the basis of the values currently stored in the Memory (7), calculates the new time rate (C1) and the new threshold (S1).

The Control Unit (1) will continue to operate as described by adjusting the values (Ci) and (Si) on the basis of the collected reponse times (Ti), (Ti - 1) (Ti - 2) ...

The logic that the Control Unit (1) uses in order to elaborate the values (Ci) and (Si), can be determined by the specific application of the system. In the presently preferred application, which is illustrated in the following paragraph with the sole purpose of exemplification without any limitation for other possible solutions, the values (Ci) and (Si) are calculated on the basis of the response times (Ti), (Ti - 1), (Ti - 2) etc, by mean of the following logic:
- At system initialization, (C0) is preset to a default value and (S0) is preset to a value which is a certain percentage v% of (C0);
- A calibration cycle is carried on, during which the Alarm Unit (3) is activated for m times, at constant intervals (CO), and corrispondently, m operator's responses are collected;
- The m values Ti, Ti -1, Ti - 2, .... Ti - m + 1 are stored in the Memory (7);
- On the basis of the m values currently stored in the Memory (7), the average value (Pi) among the last p values and the average value (Qi) among the last q values are calculated, where q = k x p and k= > 2;
- In case (Qi) is lowerer than (Pi) (the trend of the response time is growing), the interval (Ci) between two subsequent interrogations is reduced by a certain percentage s% of its current value and, in any case, the reduction will be at least t seconds;
- Contrarily, if (Qi) is greater than or equal to (Pi) (the trend of the response time is lowering or stable), the interval (Ci) between to subsequent requests is incremented by t seconds;
- the threshold (Si) is calculated as a percentage v% of (Ci), for each (Ci) obtained with the above mentioned process;
- Any time that the response time Ti + 1 is greater than (Si), the system will output an alarm.

In case an alarm condition occurs the Control Unit (1) persistently enables the sound/visual alarm and activates the Actuator (8) in order to prevent the potential negative consequences of the operator's reduced attention.

In the present preferred realization, which is illustrated in the following paragraph with the sole purpose of exemplification without any limitation for other possible solutions, the Control Unit (1) is made by a microprocessor device connected to the Memory (7) and the Timer (4) through a data bus (D), an address bus (E) and two signals, respectively: the data reading strobe (F) and the data writing strobe (G).
The Control Unit (1) receives a time base signal (H) from the Clock (2) and an attention signal (C) from the Timer (4). The same Control Unit (1) sends to the Alarm Unit (3) and to the Timer (4) the enable signal (B) and sends to the Actuator (8) the emergency signal (I). The Alarm Unit (3) is made by an electronic circuit able to generate both the interrogation signals and the alarm condition signal. The Control Unit (1) will be able to set the Alarm Unit (3), in order to generate one of the two signals, by mean of different time modulations of the enable signal (B). In the present preferred realization, the signal (B) can be modulated in three different ways: the first one, for generating through the Loudspeaker (9) a brief beep signal in order to request an "operator response", the second one for generating a persistent sound alarm and the third one for enabling the Visual Indicator (10). In an other realization, the Alarm Unit (3) is made by a voice synthesizer capable of providing a number of different vocal sentences (advices or requests) which can be selected by the proper modulation of the signal (B).
The Detector (6) is made by an electronic circuit coupled (capacitively or inductively) with the Response Circuit (5a, 5b, ..... 5n), capable to generate a response signal (A) every time a voltage or current change is detected in one of the n response circuits. The signal (A) is interpreted by the Timer(4) as an operator response to a request generated through the Alarm Unit (3).
The Timer (4) is made by an electronic circuit that receives from the Clock (2) the signal (H). This signal is exploited as time base for measuring the time interval between a response request provided by the signal (B) and the relevant operator's response provided by the signal (A). As soon as the Timer (4) has determined the value of the measured time interval, the Control Unit (1) receives the attention signal (C) which communicate to the same Controller (1) to read the value by means of the data bus (D), the address bus (E) and the data reading strobe (F).

In the present preferred \realization, the invention has been designed in order to be optimized for monitoring the level of the attention of motor vehicle human drivers. Such a realization, which is reported with the sole purpose of exemplification without any limitation for other possible solutions, uses a conventional Loudspeaker (9) and a Visual Indicator (10), made by a LED (Light Emitting Diode) group. As possible Response Circuits (5a, 5b, .... 5n) are utilized the following original vehicle circuits, which can be operated by the driver after a system response request: the head light switch, the horn button or the break pedal.

In an other possible realization, which is indicated with the sole purpose of exemplification, the invention makes use of an integrated circuit that accomplishes the functions of Control Unit (1), Clock (2), Alarm Unit (3), Timer (4), Detector (6) and Memory (7), while the remaining functional components: the transducers for coupling the Response Circuits (5a, 5b, .... 5n) with the integrated circuit, the Loudspeaker (9) and the Visual Indicator (10), are located outside the integrated circuit itself.

## Claims

**Claim #1.** The system for the automatic monitoring and the measurement of the psycho-physical level of the attention of a human operator is CHARACTERIZED BY THE FOLLOWING FACTS: A Control Unit, which exploits a time base signal provided by a Clock, periodically requests the attention of the operator by means of an Alarm Unit and measures,by means of a Timer, the time elapsed for the operator to activate one of n possible Response Circuits. The operator's response is detected by a Detector and the elapsed time interval is compared with the time intervals previously stored in a Memory. Each time the Timer measures a time interval greater than a preset threshold, the Control Unit activates an Alarm Unit that generates an acoustical and/or a visual alarm, respectively by means of a Loudspeaker and a Visual Indicator. At the same time, the Control Unit can also activate an Actuator.

**Claim #2.** The system characterized by Claim #1 is CHARACTERIZED BY THE FOLLOWING FACTS: Whether the system is used for monitoring and measuring the level of the attention of motor vehicle human drivers, the acoustical signals are generated by a Loudspeaker installed in the cabin of the vehicle.

**Claim #3.** The system characterized by Claim #1 is CHARACTERIZED BY THE FOLLOWING FACTS: Whether the system is used for monitoring and measuring the level of the attention of motor vehicle human drivers, the visual signals are generated by LED (Light Emitting Diode) group installed in the cabin of the vehicle.

**Claim # 4.** The system characterized by Claim #1 is CHARACTERIZED BY THE FOLLOWING FACTS: Whether the system is used for monitoring and measuring the level of the attention of motor vehicle human drivers, the Response Circuits which can be activated by the driver are the following original vehicle circuits: the head light switch, the horn button or the break pedal.

**Claim # 5.** The system characterized by Claim #1 is CHARACTERIZED BY THE FOLLOWING FACTS: The following functional components: Control Unit, Clock, Alarm Unit, Timer, Detector and Memory are included in a single integrated circuit, while the transducers for coupling the Response Circuits with the integrated circuit, the Loudspeaker and the Visual Indicator, are located outside the integrated circuit itself.

**Claim # 6.** The system characterized by Claim #1 is CHARACTERIZED BY THE FOLLOWING FACTS: The Alarm Unit is made by an electronic devices capable of generating a number of pre-stored vocal sentences, which can be singularly selected and activated by means of a signal sent to the Alarm Unit by the Control Unit itself.

**Claim # 7.** The system characterized by Claim #1 is CHARACTERIZED BY THE FOLLOWING FACTS: The alarm threshold (S) is continuously adjusted according to the operator's response time, with such a logic that the system increases its surveillance action (increasing the frequecy of the response requests) when the operator's response times increases.

**Claim # 8.** The system characterized by Claim #1 and claim #7 is CHARACTERIZED BY THE FOLLOWING FACTS: The initial alarm threshold is set by the system to a value which correspond to a pre-defined fractional value of the last two measured response times.

**Claim # 9**. The system characterized by Claim #1 and claim #7 and Claim #8 is CHARACTERIZED BY THE FOLLOWING FACTS: The operator's response times are stored in a Memory capable to store a pre-determined number of values which correspond to the most recent response times.

**Claim #10.** The system characterized by Claim #1 and Claim #7 and Claim #8 and Claim #9 is CHARACTERIZED BY THE FOLLOWING FACTS: The average value of the response times stored in the Memory is continuously compared with the response time relevant to the most recent operator's response. The result of this comparison is used in order to increase or to decrease the alarm threshold.
